# EUROPEAN PATENT APPLICATION

(11) **EP 3 321 352 A1**
(43) Date of publication of application: **16.05.2018**
(21) Application number: 16197889.5
(22) Date of filing: 09.11.2016
(51) Int. Cl.: C12N 1/02, C12Q 1/04, B01L 3/00, C12M 3/06, C12M 1/12, C12M 1/42, C12M 1/00

(54) **AUXOTROPHIC SELECTION SYSTEM**

(71) Applicant: Biomillenia SAS, 75005 Paris (FR)
(72) Inventor: Löffert, Dirk, 42781 Haan (DE)
(74) Representative: CH Kilger Anwaltspartnerschaft mbB

(57) **Abstract**

A method for the analysis of microorganisms, which produce a compound, the method comprising:
a. Providing a microorganism which produces a compound of interest and a detector microorganism which comprises a reporter gene or reporter gene operon; wherein the microorganism producing said compound of interest and the detector microorganism are combined into single droplets, wherein each droplet comprises at least one cell of each strain;
b. subjecting the droplets to a microfluidic system;
c. analyzing the droplets for the activation of the reporter gene of the detector strain;
d. sorting and collecting the droplets comprising the detector microorganism with expressed reporter gene.

## Description

### Field of the invention

The present application is in the field of cell culture analysis. More precisely in the field of cell culture analysis on single cell level. The application is also in the field of microfluidics, in particular in the field of microfluidic analysis and devices.

### Background

The production of biological compounds such as sugars, amino acids, antibiotics, carbon sources or nitrogen sources and other chemical building blocks today is often efficiently performed in microorganisms. With the tools of genetic engineering it is possible to optimize microorganisms for an increased production of compounds.

These optimized microorganisms are generated using different mutagenic/combinatorial strategies capable to generate large libraries of genetically modified organisms. However, the drawback or bottleneck of all strategies are the screening methods used to analyze individual library members.

The relevant screening methods are dependent on the molecules to be produced, but commonly the screening methods are based on chromatography and subsequent detection, in many cases by mass spectroscopy. A great disadvantage of this is that parallelization and high throughput is difficult to achieve, as the number of clones that can be analyzed is limited.

Accordingly, there is a need for new screening methods, which allow the detection of strains, which show improved properties in the production of compounds, in particular small molecules such as amino acids or sugars or intermediate chemical building blocks.

One approach was the use of biosensors for the analysis or identification of small molecules in production media. Pfleger et al. (Pfleger BF, et al.; Metabolic Engineering; 2007; 30-38) describe the generation of a *E. coli* strain, which is suitable as mevalonate biosensor and expresses GFP in the presence of mevalonate, allowing quantitative detection of mevalonate in an extracellular environment.

Bertels, et al. (Bertels, F. et al.; PLoS ONE; 2012; e41349) describe the development of a biosensor for amino acids, based on an auxotrophic *E. coli* strain comprising the eGFP gene. US 9,279,139 B2 describes an *E. coli* glutamine biosensor, comprising the *lux* operon.

However, all of these methods are still limited, as they do not allow the rapid analysis of large libraries of colonies. There is therefore still a need for an improved screening method, which allows high throughput screening of microorganism libraries.

### Brief description of the invention

The present invention aims to solve this problem by combining the traditional screening approaches with microfluidic devices, thus breaking down the analysis onto single cell level instead of cell cultures.

The invention relates to a method for the analysis and/or selection of microorganisms, preferably microorganisms which produce a compound of interest, the method comprising:
a. Providing a microorganism which produces a compound of interest and a detector microorganism which comprises a reporter gene or reporter gene operon; wherein the microorganism producing said compound of interest and the detector microorganism are combined into single droplets, wherein each droplet comprises at least one cell of each strain;
b. subjecting the droplets to a microfluidic system;
c. analyzing the droplets for the activity of the reporter gene of the detector strain;
d. sorting and collecting the droplets comprising the detector microorganism with the active reporter gene.

The invention further relates to the use of the method for the analysis of a mutated microorganism, producing a compound.

In a further aspect the invention relates to a microfluidic device capable of co-encapsulating at least two types of cells, the device comprising:
a) at least one inlet for a culture medium comprising a first microorganism;
b) at least one inlet for a culture medium comprising a second microorganism;
c) optionally at least one inlet for an immiscible phase;
d) a chamber for combining the first and second medium, suitable to generate droplets comprising at least one cell of each microorganism, and optionally to encapsulate the droplets in the immiscible phase;
e) optionally means to incubate the droplets at a constant or variable temperature;
f) optionally a detector to detect the activity of a reporter gene;
g) optionally means for sorting the droplets and an outlet for sorted droplets.

The invention further relates to the use of said microfluidic device.

### Detailed description of the invention

The present invention relates to a method for the analysis of microorganisms for improved properties. The invention in particular relates to screening methods for microorganisms, which are able to produce biological compounds. In contrast to other screening methods the claimed screening method allows analysis on single cell level.

The invention further relates to microfluidic devices suitable for performing the method.

In a first aspect the invention relates to a method for the analysis and/or selection of microorganisms, which produce a compound, the method comprising:
a. Providing a microorganism which produces a compound of interest and a detector microorganism which comprises a reporter gene or reporter gene operon; wherein the microorganism producing said compound of interest and the detector microorganism are combined into single droplets, wherein each droplet comprises at least one cell of each strain;
b. subjecting the droplets to a microfluidic system;
c. analyzing the droplets for the activity of the reporter gene of the detector strain;
d. sorting and collecting the droplets comprising the detector microorganism with active reporter gene.

In the context of the present invention an activated reporter gene or the activity of the reporter gene refers to the expression of a detectable gene product. Said gene product might be continuously expressed or the expression might be triggered under certain conditions.

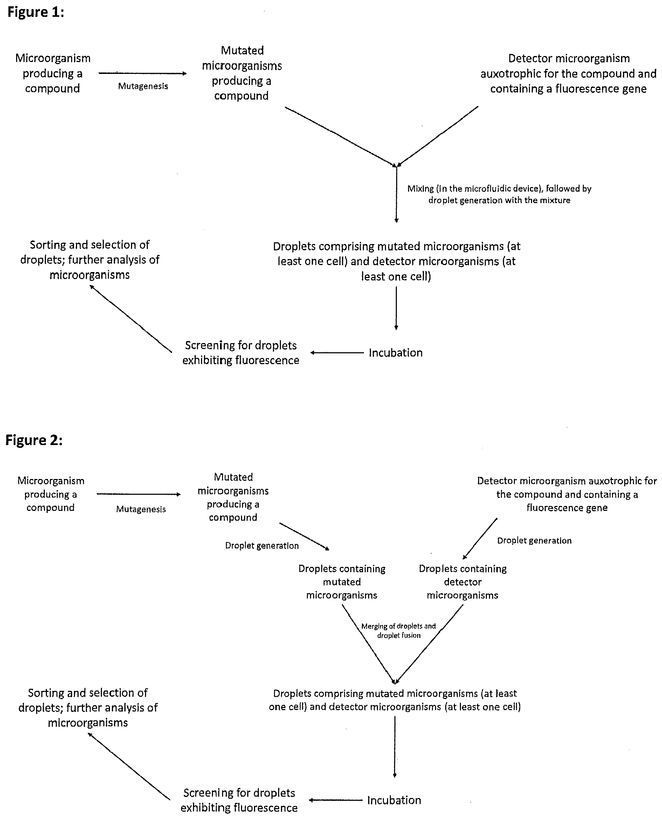

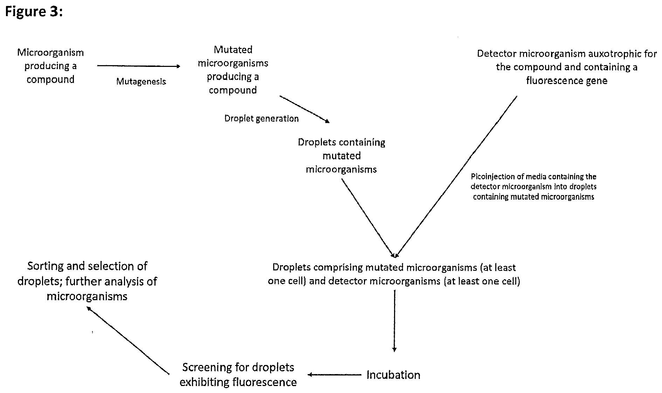

A general schematic of preferred workflows of the method is shown in figures 1 to 3.

The method is suitable for any kind of microorganism, which can be handled on single cell level. The microorganism, which produces a compound and the detector microorganism might be of the same species or different species.

The method is particularly suitable for the analysis of microorganisms, which had been mutated or genetically engineered in order to optimize the production of desired compounds. In one embodiment of the invention the microorganism, which produces a compound is therefore a mutated or genetically engineered organism.

Mutated or genetically engineered organisms can be generated by means known to the person skilled in the art. Sample methods to induce mutations in microorganisms include but are not limited to, exposure to radiation, in particular UV-radiation or radioactive radiation, stress, phages and viruses, transposon mutagenesis, homologous recombination, metabolic engineering, or chemical mutagenesis. Alternatively, the microorganism producing a compound may comprise a plasmid or cosmid comprising a modified or mutated enzyme or biosynthesis pathway.

Suitable microorganisms, which might be mutated or produce a compound include, but are not limited to bacterial strains, archeal strains, fungal strains, yeast strains, algae, plant protoplasts, prokaryotic or eukaryotic cells, spores, insect cells or insect strains. In a preferred embodiment of the invention the microorganism which produces a compound of interest is a bacterial strain, a fungal strain or yeast strain. In a most preferred embodiment the microorganism, which produces a compound, is a bacterial or fungal strain.

In a preferred embodiment of the invention, a library of microorganisms producing a compound of interest is generated and analyzed. The method of the invention is in particular suitable for screening for microorganisms exhibiting a higher productivity of the compound and a higher final titers of the compound, in a library of microorganisms.

The produced compound of interest might be any compound, which can be exported or secreted into the medium by the microorganism and which can be detected by a detector microorganism. The compound preferably has either direct commercial value or may serve as an intermediate in the production of a further compound, which has commercial value.

Suitable compounds include, but are not limited to, primary metabolites: L- and D- amino acids; sugars and carbon sources such as L-arabinose, N-acetyl-D-glucosamine, N-acetyl-D-mannosamine, N-acetylneuraminate, lactose, D-glucosamine, D-glucose-6-phosphate, D-xylose, D-galactose, glycerol, maltose, maltotriose, and melibiose; nucleosides such as cytidine, guanine, adenine, thymidin, guanosine, adenosine; lipids such as hexadecanoate and glycerol 3-phosphate; indole, maltohexose, maltopentose, putrescine, spermidine, ornithine, tetradecanoate, and nicotinamide adenine dinucleotide..

Further relevant compounds of interest include, but are not limited to, secondary metabolites. Such metabolites can be produced naturally by the producer microorganism but may also be generated via a heterologous biosynthetic pathway introduced into the microorganisms by genetic engineering. Examples of secondary metabolites include, but are not limited to, polyketides (such as erythryomycin and avermectins), small molecules (such as resveratrol, steviol, and artemisenin) or non-ribosomal peptides.

The detector microorganism may also be any organism that can be handled on single cell level. Suitable microorganisms, which might be mutated or genetically engineered, include, but are not limited to, bacterial strains, archeal strains, fungal strains, yeast strains, algae, plant protoplasts, prokaryotic or eucaryotic cells, spores, insect cells or insect strain.

Preferably, the detector strain is a different microorganism than the strain producing a compound. More preferably, the detector strain is a bacterial strain. Most preferably the detector strain is an *E. coli strain.*

The detector strain has to comprise a reporter gene or a reporter gene operon. Preferably, said reporter gene or reporter gene operon produces a detectable signal. In one embodiment, the intensity of said detectable signal correlates with the amount of produced compound. In an alternative embodiment the intensity of said signal is independent of the amount of compound produced.

In a preferred embodiment the detectable signal is a fluorescent signal. In one embodiment, said fluorescent signal is generated by the reporter gene product or the reporter gene operon. In a preferred embodiment the reporter gene encodes a fluorescent protein such as green fluorescent protein (GFP), a variant of GFP, yellow fluorescent protein (YFP), a variant of YFP, red fluorescent protein (RFP), a variant of RFP, cyan fluorescent protein (CFP), a variant of CFP or the reporter gene operon is a luminescence operon such as the lux operon. It is known to the person skilled in the art that homologs of said proteins may be used.

Preferably the detector strain is an *E. coli* strain. In a more preferred embodiment the detector strain is a mutated *E. coli* strain, optimized for the detection of the compound. *E. coli* strains can be easily mutated by standard and well known techniques.

There are two general possibilities for the detection of the compound of interest. In a first embodiment of the invention, the reporter gene or reporter gene operon of the detector strain might be activated in the presence of said compound. A possible example would be a modified lac-operon, which is utilized for protein expression. Depending on the compound and organism, several potential operons suitable are known for the person skilled in the art. In general, suitable operons usually trigger the degradation or metabolization of the compound.

One further possibility is the use of modified allosteric transcription factors as described by *Taylor er al.* (Taylor, N.D, et al.; Nature Methods, Vol. 13; 2016; 177-183) or the use of synthetic biosensors as described by Rogers *et al.* (Rogers, J. K. et al.; Nucleic Acids Research; 43; 2015; 7648-7660).

An alternative preferred detector strain might be auxotrophic for the compound, i.e. the detector strain cannot survive without an exogenous supply of said compound. In this case the reporter gene might be continuously activated.

A detector microorganism which is auxotrophic for compound A is unable to grow unless compound A is present in the culture medium. Such a microorganism could be generated via knockout of one or more genes in said microorganism. In the absence of these genes, the microorganism would be unable to synthesize compound A. In some cases, compound A is required directly for growth. In other cases, compound A serves as an intermediate for the synthesis of compound B, which is required for growth. Preventing the synthesis of compound A therefore precludes the synthesis of compound B and prevents cell growth.

Methods to generate auxotrophic microorganisms are known to the person skilled in the art. Suitable methods include the generation of knockout mutants or random mutagenesis.

Alternatively, several naturally existing microorganisms are auxotrophic for specific compounds. In most cases said microorganisms are auxotrophic for amino acids.

If genome-scale models are available, the compounds which may be sensed and the corresponding gene knockouts which must be made to achieve auxotrophy may be determined based on a computational optimization problem formulated around the available genome-scale model (e.g., Tepper et al (2011), "Computational design of auxotrophy-dependent microbial biosensors for combinatorial metabolic engineering experiments" PLOS ONE 6:1).

Gene knockouts may be achieved via a variety of methods, including but not limited to homologous recombination, gene inactivation via PCR products (e.g., Datsenko and Wanner (2000), "One-step inactivation of chromosomal genes in Escherichia coli K-12 using PCR products" PNAS 97(12):6640-6645), CRISPR-Cas9, transposon mutagenesis, and phage transduction. Thus, auxotrophic sensor strains can be generated today with little effort and time required.

Generated auxotrophic microorganisms may also be engineered to express a reporter molecule, which may be a fluorescent protein (green fluorescent protein or its derivatives such as eGFP, red fluorescent protein or its derivatives such as mCherry, cyan fluorescent protein or its derivatives, yellow fluorescent protein or its derivatives) or an operon of genes whose expression results in luminescence (such as the *lux* operon). In a preferred embodiment generated auxotrophic microorganisms are also engineered to express a reporter molecule, which may be a fluorescent protein (green fluorescent protein or its derivatives such as eGFP, red fluorescent protein or its derivatives such as mCherry, cyan fluorescent protein or its derivatives, yellow fluorescent protein or its derivatives) or an operon of genes whose expression results in luminescence (such as the *lux* operon).

The cultivation of microorganisms is known to the person skilled in the art. In general, microorganisms are cultivated in a liquid medium or on a solid medium. In general solid media are based on liquid media.

Prior to analysis the cells might be cultured in any suitable culture medium. Suitable culture media are dependent on the microorganisms. The person skilled in the art generally differentiates between undefined media, such as for example LB-medium, and defined media, in particular minimal media, such as M9 minimal medium or MOPS minimal medium.

Undefined media usually comprise water, a carbon source, a protein and nitrogen source and salts. In general, the carbon, protein and nitrogen source can be an extract, for example yeast and/or beef extract or protein hydrolysates, such as tryptone or peptone. The exact amino acid composition and salt concentration or composition is usually unknown.

Defined media on the other hand are exactly known. In a defined medium, all used chemicals are known and the concentrations of the other compounds are known. In the specific case of minimal media, the medium contains the minimum nutrients possible for colony growth, generally without the presence of amino acids.

As not every organism is able to grow in any medium, it is necessary to adapt the selected medium to the types of microorganisms used. For analysis, a medium which allows survival of both microorganisms is necessary. Depending on the selected microorganisms the person skilled in the art will know and be able to select the right growth medium.

Accordingly, the method is not suitable for every combination of microorganisms. It is for example not possible to cultivate a microorganism requiring a medium with high salt concentration together with a microorganism requiring a low salt concentration. Therefore, the detector microorganism needs to be selected dependent on the microorganism producing a compound.

Preferably, prior to the analysis according to the method of the invention, the microorganisms are cultivated separately in appropriate media. In one embodiment of the invention the microorganisms are cultivated and incubated in full media. In an alternative embodiment the microorganisms are cultivated in defined media, preferably minimal media.

In an alternative embodiment of the invention, the microorganism producing a compound is cultivated in a full medium and the detector microorganism is cultivated in a defined medium, preferably cultivated in a minimal medium.

For analysis the microorganisms are then used in their respective medium or transferred in an analysis medium. Preferably, said analysis medium is a defined medium. In a more preferred embodiment said analysis medium is a minimal medium.

The person skilled in the art knows how to transfer cell cultures in different media. In one embodiment the different culture media are simply mixed to form a new culture medium. In a preferred embodiment the microorganisms are transferred using several centrifugation and washing steps, involving suspending the cells in the target medium.

Microorganisms in the analysis media are then diluted and/or encapsulated into single droplets. Droplet generation is known to the person skilled in the art. Preferably, said droplets are generated using a microfluidic device. Preferably, during droplet generation the microorganism producing a compound and the detector microorganism are combined. Alternatively, the microorganism producing a compound and the detector strain are diluted into separate droplets and two droplets, each comprising one of the microorganisms are united into a single droplet.

Regardless of the method of droplet generation it is preferred that the final droplets in their majority comprise at least one microorganism of each type, i.e. at least one microorganism producing a compound and at least one detector microorganism. Preferably, the majority of droplets comprises one cell of each microorganism.

It is essential that the droplets additionally comprise all necessary compounds to support growth of the microorganisms, both the detector microorganism and the microorganism producing a compound, and to support the production of said compound by the producing microorganism.

The droplets comprising the microorganisms may be additionally encapsulated to separate the contents from the environment. A possible method of encapsulation is discussed in WO 2010/063937 A1. In a preferred embodiment the droplets are encapsulated in a soft alginate shell.

Alternatively, the droplets are separated from the environment using a phase immiscible with the medium to separate or encapsulate droplets. In one embodiment said immiscible phase is an oil. In a more preferred embodiment said immiscible phase is a fluorinated oil.

In one embodiment the droplets have a volume of between 1pL and 1µL.

After diluting and optionally encapsulating the droplets, the microorganisms are incubated for an appropriate amount of time. Said incubation might be performed directly in the microfluidic device or separate from the microfluidic device.

Incubation might be performed in any way possible. It is however important that the droplets remain intact during the incubation. Stable droplets might be incubated outside of a microfluidic device and later again be subjected to a microfluidic device.

Independently from where the droplets and microorganisms are incubated it is preferred that the microorganisms are incubated at appropriate temperatures. The suitable temperature is dependent on the microorganisms in the droplets and the requirements for the production of the compounds. For example, bacterial cultures, such as E. coli usually require temperatures between 20 and 37 °C.

In one embodiment the incubation temperature is between 18 °C and 50 °C; In a preferred embodiment the incubation temperature is between 20 and 48 °C. In a more preferred embodiment the incubation temperature is between 25 and 45 °C. In an even more preferred embodiment the incubation temperature is between 35 and 40 °C. In the most preferred embodiment the incubation temperature is 37 °C.

The temperature may vary during incubation or may be constant. In one embodiment of the invention the droplets comprising the microorganisms are incubated at a constant temperature. In an alternative embodiment the droplets comprising the microorganisms are incubated at variable temperatures.

Incubation time has to be selected accordingly. In general, the incubation time needs to be long enough to allow for the microorganisms to grow and produce and detect the compound. The time is dependent of the medium, the temperature and the microorganisms. A "richer" medium and a temperature near the optimum temperature for the microorganism results in shorter incubation times.

After incubation the droplets are analyzed in a microfluidic device, screening for the activation of the reporter gene. The detection method is dependent on the reporter gene. If the reporter gene is a fluorescent protein or a reporter operon generating a fluorescent signal, the detection method is fluorescence detection.

Preferably, following incubation, the concentration of the reporter molecule in each droplet is determined via fluorescence or luminescence measurements. Such measurements may be performed on the same microfluidic device in which the droplets were generated or on a second microfluidic device distinct from the first microfluidic chip. Preferably, improved production strains can be identified by fluorescence or luminescence above that measured from droplets produced by co-encapsulating the biosensor strain with the parent production strain.

After detection the droplets which had been identified as comprising an activated reporter gene or a surviving detector microorganism are selected and separated for further analysis. Potential mechanisms for sorting the droplets are known to the person skilled in the art. In one embodiment the cells are sorted using dielectrophoresis.

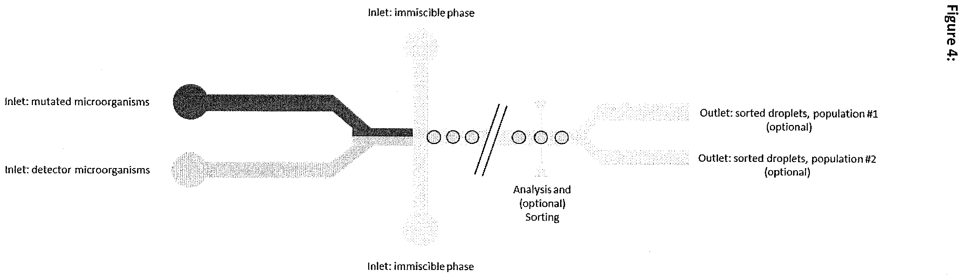

The invention also relates to several devices to be used in said method. In a first aspect the invention relates to a microfluidic device capable of co-encapsulating at least two types of cells, the device comprising (see figure 4):
a) at least one inlet for a culture medium comprising a first microorganism;
b) at least one inlet for a culture medium comprising a second microorganism;
c) at least one inlet for a phase immiscible with the culture media;
d) a chamber for combining the first and second medium, suitable to generate droplets comprising at least one cell of each microorganism, and optionally to encapsulate the droplets in the immiscible phase.

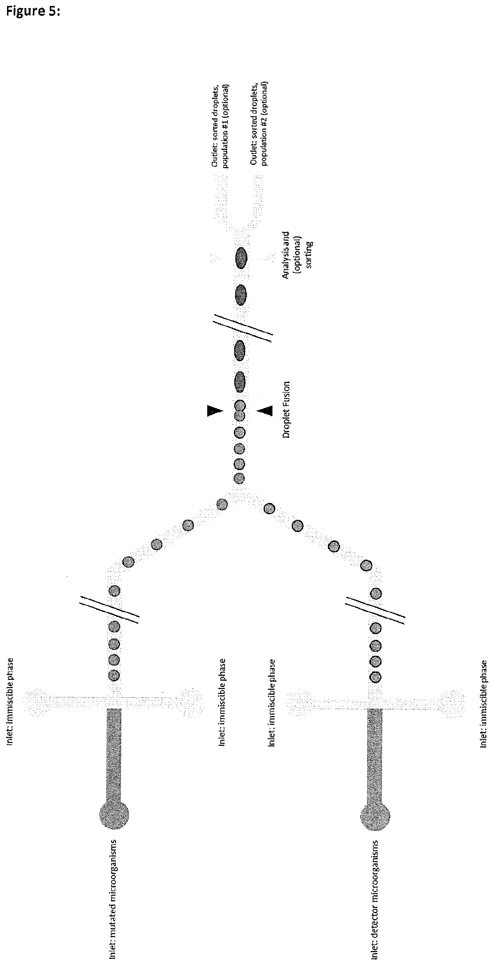

In an alternative embodiment the invention relates to a microfluidic device capable of co-encapsulating at least two types of cells, the device comprising (see figure 5):
a) at least one inlet for a culture medium comprising a first microorganism;
b) at least one inlet for a culture medium comprising a second microorganism;
c) at least one inlet for a phase immiscible with the culture media;
d) a chamber for combining the first and second medium, suitable to generate droplets comprising at least one cell of each microorganism, and optionally to encapsulate the droplets in the immiscible phase.

In a further embodiment the invention relates to a microfluidic device capable of co-encapsulating at least two types of cells, the device comprising:
a) a chamber for generating droplets of the first medium, and to encapsulate the droplets in the immiscible phase;
b) a chamber for generating droplets of the second medium, and to encapsulate the droplets in the immiscible phase;
c) a chamber for combining droplets of the first medium with droplets of the second medium and subsequently fusing said droplets to yield larger droplets comprising a mixture of the first medium and the second medium.

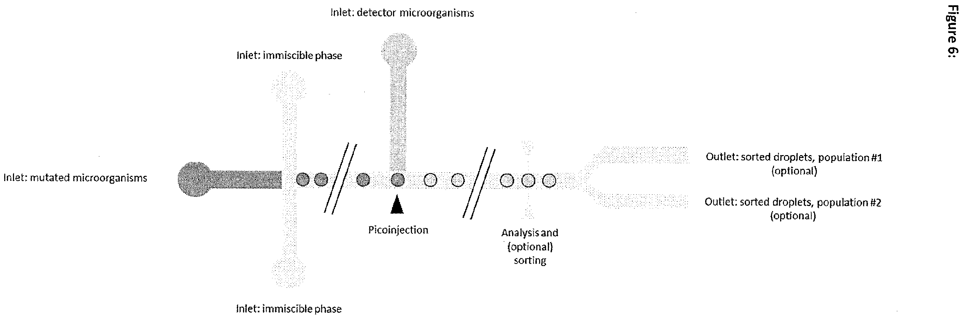

In one embodiment the droplets comprising at least one cell of each medium are generated by generating a droplet comprising at least one cell of a first microorganism and in said chamber picoinjecting said second microorganism into said droplet (see figure 6).

In an alternative embodiment the droplets are generated by generating droplets comprising a first microorganism and droplets comprising the second microorganism and in the chamber combining and/or fusing the droplets into single droplets.

The microfluidic device may optionally comprise further inlets. Said inlets might be for further modifications of the droplets, e.g. for adding additional components into the culture medium. Alternatively, said additional inlets might be used for modification of the droplets such as mixing of droplets, addition of other droplets into the stream for subsequent fusion, addition of spacing oil to further separate droplets the droplets.

Said microfluidic device might be a standalone device, or part of a larger microfluidic device. If said microfluidic device is a standalone device, it is preferred that the device can be connected to other devices, preferably other microfluidic devices.

In one embodiment the microfluidic device comprises means for temperature control in order to maintain the culture media comprising microorganisms at a desired temperature. Preferably, the microfluidic device comprises means for temperature control in order to maintain the culture media comprising the microorganisms at constant temperature. More preferably, the microfluidic device comprises means for temperature to maintain the culture media comprising the microorganisms at a constant temperature during the whole droplet generation process.

In a preferred embodiment the microfluidic device allows the control of droplet size. In a more preferred embodiment, the microfluidic device allows for the generation of droplets with variable size. In the most preferred embodiment, the microfluidic device allows for the generation of a monodisperse population of droplets.

Optionally, the microfluidic device comprises means for a further treatment of the droplets, such as additional means for injection of reagents, injection of cells, temperature control, delay lines for on chip incubation, sorting of droplets.

In a further aspect the invention relates to a microfluidic device capable of co-encapsulating at least two types of cells, the device comprising:
a) at least one inlet for a culture medium comprising a first microorganism;
b) at least one inlet for a culture medium comprising a second microorganism;
c) optionally at least one inlet for a phase immiscible with the culture media;
d) a chamber for combining the first and second medium, suitable to generate droplets comprising at least one cell of each microorganism, and optionally to encapsulate the droplets in the immiscible phase;
e) optionally means to incubate the droplets at a constant or variable temperature;
f) optionally a detector to detect the activity of a reporter gene;
g) optionally an outlet coupled with means for sorting droplets.

In one particular embodiment the invention relates to a microfluidic device for generating, incubating and analyzing and/or sorting droplets comprising cells, the device comprising:
a) a first inlet for a culture medium comprising a first microorganism;
b) a second inlet for a culture medium comprising a second microorganism;
c) a third inlet for a phase immiscible with the culture media;
d) a chamber for combining the first and second medium, suitable to generate droplets comprising at least one cell of each microorganism, and to encapsulate the droplets in the oil;
e) optionally means to incubate the droplets at a constant or variable temperature;
f) a detector to detected the activity of a reporter gene;
g) an outlet coupled with means for sorting droplets.

In a particular embodiment the invention relates to a microfluidic device for generating, incubating and sorting droplets comprising cells, the device comprising:
a) a first inlet for a culture medium comprising a first microorganism;
b) a second inlet for a culture medium comprising a second microorganism;
c) a third inlet for an oil;
d) a chamber for combining the first and second medium, suitable to generate droplets comprising at least one cell of each microorganism, and to encapsulate the droplets in the oil;
e) optionally means to incubate the droplets at a constant or variable temperature;
f) optionally a detector to detected the activity of a reporter gene;
g) optionally an outlet coupled with means for sorting droplets.

In another particular embodiment the invention relates to a microfluidic device for generating, incubating and sorting droplets comprising cells, the device comprising:
a) a first inlet for a culture medium comprising a first microorganism;
b) a second inlet for a culture medium comprising a second microorganism;
c) a third inlet for an oil;
d) a fourth inlet for an oil;
e) a chamber for encapsulating droplets of the first medium in the oil;
f) a chamber for encapsulating droplets of the second medium in the oil;
g) a chamber for combining droplets of the first medium with droplets of the second medium and fusion of droplets into larger droplets comprising a mixture of the first medium and second medium;
h) optionally a means to incubate the droplets at a constant or variable temperature;
i) optionally a detector to detected the activity of a reporter gene;
j) optionally an outlet coupled with means for sorting droplets.

In another particular embodiment the invention relates to a microfluidic device for creating incubating and sorting droplets comprising cells, the device comprising:
a) a first inlet for a culture medium comprising a first microorganism;
b) a second inlet for a culture medium comprising a second microorganism;
c) a third inlet for an oil;
d) a fourth inlet for an oil;
e) a chamber for encapsulating droplets of the first medium in the oil;
g) a chamber for combining droplets of the first medium with the second medium via picoinjection, generating larger droplets comprising a mixture of the first medium and second medium;
h) optionally a means to incubate the droplets at a constant or variable temperature;
i) optionally a detector to detected the activity of a reporter gene;
j) optionally an outlet coupled with means for sorting droplets.

The microfluidic device according to the invention preferably comprises at least three inlets, one for a culture medium comprising a first microorganism, which is preferably producing a compound, one for a second culture medium, comprising a second microorganism, which comprises a reporter gene or reporter gene operon and a third inlet for an immiscible phase.

Said immiscible phase might be an oil or a gas. Preferably, said immiscible phase is a oil, preferably fluorinated oil. In an alternative preferred embodiment said immiscible phase is an gas.

In one embodiment the microfluidic device comprises means for temperature control in order to maintain the culture media comprising microorganisms at a desired temperature. Preferably, the microfluidic device comprises means for temperature control in order to maintain the culture media comprising the microorganisms at constant temperate. More preferably, the microfluidic device comprises means for temperature to maintain the culture media comprising the microorganisms at a constant temperature during the whole droplet generation process.

In a preferred embodiment the microfluidic device allows the control of droplet size. In a more preferred embodiment, the microfluidic device allows for the generation of droplets with variable size.

In a particular embodiment the microfluidic device allows to incubate the droplets. Means for incubation are known to the person skilled in the art. A possible way would be a temperature controlled loop, which allows temperature controlled incubation.

In a preferred embodiment, the device allows to control incubation temperature. In one embodiment of the invention, the microfluidic device allows incubation at a constant temperature. In an alternative embodiment the device allows incubation at a variable temperature.

In an alternative embodiment the microfluidic device comprises an outlet and/or an additional inlet, allowing to remove the droplets for incubation and to reinsert the droplets into the microfluidic device. In one embodiment, at least two ports may be used, one inlet and one outlet. In an alternative embodiment, one port may serve as inlet and outlet.

The device preferably comprises means for the detection of the activation of the reporter gene. As it is preferred that the reporter gene provides a fluorescent signal, said means preferably allow the detection of fluorescence and more preferably, additional determination of fluorescence intensity.

In a preferred embodiment the detector is coupled to a computing device.

Finally the device preferably comprises, optionally an outlet, which allows sorting the droplets. Preferably, the outlet allows the sorting of droplets showing increased fluorescence compared to other droplets. In a preferred embodiment said outlet comprises means which allow sorting via dielectrophoresis.

In a preferred embodiment said outlet is coupled to a computing device.

The invention further relates to a microfluidic device for the analysis of droplets comprising single cells, preferably single cells of each one microorganism producing a compound and a detector microorganism. The microfluidic device comprises:
a) an inlet for droplets;
b) optionally means for maintaining the droplets at a defined temperature;
c) a detector to detect the activity of a reporter gene;
d) at least one outlet.

In a preferred embodiment, the device allows to control temperature. In one embodiment of the invention, the microfluidic device allows to keep the droplets at a constant temperature. In an alternative embodiment the device allows to keep the droplets at a variable temperature.

The device comprises means for the detection of the activation of the reporter gene; As it is preferred that the reporter gene provides a fluorescent signal, said means preferably allow the detection of fluorescence and more preferably, additional determination of fluorescence intensity..

In a preferred embodiment the detector is coupled to a computing device.

The device preferably comprises at least one outlet. Preferably, the outlet allows the sorting of droplets. In a preferred embodiment said outlet allows sorting of droplets via dielectrophoresis.

In a preferred embodiment said outlet is coupled to a computing device.

### Figure legends:

Figures 1 to 3: schematic examples of preferred workflows of the method.
Figures 4 to 6: schematics of microfluidic devices for droplet generation. Broken lines represent positions, where the droplets might be further processed either within or of the microfluidic device.

## Claims

1. A method for the analysis of microorganisms, which produce a compound of interest, the method comprising:
a. providing a microorganism which produces a compound of interest and a detector microorganism which comprises a reporter gene or reporter gene operon; wherein the microorganism producing said compound of interest and the detector microorganism are combined into single droplets, wherein each droplet comprises at least one cell of each strain;
b. subjecting the droplets to a microfluidic system;
c. analyzing the droplets for the activity of the reporter gene of the detector strain;
d. sorting and collecting the droplets comprising the detector microorganism with expressed reporter gene.

2. A method according to claim 1, wherein the microorganism producing a compound and/or the detector microorganism is a bacterial, fungal, yeast, algal, eukaryotic, prokaryotic, or insect strain.

3. A method according to claims 1 or 2, wherein the reporter gene product produces a fluorescent signal.

4. A method according to any of the claims 1 to 3, wherein the reporter gene encodes a fluorescent protein such as green fluorescent protein (GFP), a variant of GFP, yellow fluorescent protein (YFP), a variant of YFP, red fluorescent protein (RFP), a variant of RFP, cyan fluorescent protein (CFP), a variant of CFP or the reporter gene operon is a luminescence operon such as the lux operon.

5. A method according to any of the claims 1 to 4, wherein the incubation is performed in the microfluidic system.

6. A method according to claims 1 to 5, wherein the compound is a primary metabolite, including but not limited to: L- and D- amino acids; sugars and carbon sources such as L-arabinose, N-acetyl-D-glucosamine, N-acetyl-D-mannosamine, N-acetylneuraminate, lactose, D-glucosamine, D-glucose-6-phosphate, D-xylose, D-galactose, glycerol, maltose, maltotriose, and melibiose; nucleosides such as cytidine, guanine, adenine, thymidin, guanosine, adenosine; lipids such as hexadecanoate and glycerol 3-phosphate; indole, maltohexose, maltopentose, putrescine, spermidine, ornithine, tetradecanoate, and nicotinamide adenine dinucleotide or a secondary metabolite.

7. A microfluidic device capable of co-encapsulating at least two types of cells, the device comprising:
a) at least one inlet for a culture medium comprising a first microorganism;
b) at least one inlet for a culture medium comprising a second microorganism;
c) at least one inlet for a phase immiscible with the culture media;
d) a chamber for combining the first and second medium, suitable to generate droplets comprising at least one cell of each microorganism, and to encapsulate the droplets in the immiscible phase;
e) optionally means to incubate the droplets at a constant or variable temperature;
f) optionally a detector to detect the activity of a reporter gene;
g) optionally an outlet coupled with means for sorting droplets.

8. A microfluidic device according to claim 7 capable of co-encapsulating at least two types of cells, the device comprising:
a) a chamber for generating droplets of the first medium, and to encapsulate the droplets in the immiscible phase;
b) a chamber for generating droplets of the second medium, and to encapsulate the droplets in the immiscible phase;
c) a chamber for combining droplets of the first medium with droplets of the second medium and subsequently fusing said droplets to yield larger droplets comprising a mixture of the first medium and the second medium.

9. A microfluidic device according to claim 7 capable of co-encapsulating at least two types of cells, the device comprising:
a) a chamber for generating droplets of the first medium, and to encapsulate the droplets in the immiscible phase;
b) a chamber for combining droplets of the first medium with the second medium by picoinjection to yield droplets comprising a mixture of the first medium with the second medium;

10. A microfluidic device according to any of claims 7 to 9, wherein the detector is a fluorescence detector.

11. A microfluidic device according to claim 10, wherein detector is a fluorescence detector and able to quantify the fluorescence intensity.

12. A microfluidic device according to any of the claims 7 to 11, wherein the detector is coupled to a computing device.

13. A microfluidic device according to any of the claims 7 to 12, wherein the device comprises means to incubate the droplets at a temperature range between 18 °C and 50 °C.

14. A microfluidic device according to any of claims 7 to 13, wherein the means for sorting droplets comprise dielectric sorting of droplets.

15. Use of a microfluidic device according to any of the claims 7 to 14 in a method according to claims 1 to 6.
